# EUROPÄISCHE PATENTANMELDUNG

(11) **EP 2 014 313 A2**
(43) Veröffentlichungstag der Anmeldung: **14.01.2009**
(21) Anmeldenummer: 08010106.6
(22) Anmeldetag: 03.06.2008
(51) Int. Cl.: A61L 9/04

(54) **Vorrichtung zum Verdunsten von Essenzen aufweisenden Flüssigkeiten**

(30) Priorität: 08.06.2007 DE 102007026603
(71) Anmelder: Calin, Michael, 75223 Niefern-Öschelbronn (DE)
(72) Erfinder: Calin, Michael, 75223 Niefern-Öschelbronn (DE)
(74) Vertreter: Dimmerling, Heinz

(57) **Zusammenfassung**

Eine Vorrichtung zum Verdunsten von Essenzen aufweisenden Flüssigkeiten (1a) wie ätherische Öle, mit einem Behälter (1), in den die Flüssigkeit (1a) einbringbar ist, sowie einem Verdunsterelement (2, 3), mittels dem die Flüssigkeit (1a) aus dem Behälter (1) entnehmbar und an die Umgebungsluft abgebbar ist, ist dadurch gekennzeichnet, dass das Verdunsterelement (2, 3) ein saugfähiges flächiges Element (2) aufweist.

## Beschreibung

Die Erfindung betrifft eine Vorrichtung nach dem Oberbegriff des Anspruchs 1, zum Verdunsten von Essenzen aufweisenden Flüssigkeiten wie ätherische Öle, mit einem Behälter, in den die Flüssigkeit einbringbar ist, sowie einem Verdunsterelement, mittels dem die Flüssigkeit aus dem Behälter entnehmbar und an die Umgebungsluft abgebbar ist.

Eine derartige Vorrichtung ist im Stand der Technik hinlänglich bekannt und wird regelmäßig zum Beduften von Räumen verwendet.

Bei der bekannten Vorrichtung wird beziehungsweise werden in den Behälter ein beziehungsweise mehrere Holzstäbchen eingebracht, welches beziehungsweise welche aus einem Holz bestehen, welches aufgrund seiner Struktur im Inneren eine Vielzahl von filigranen rohrförmigen Hohlräumen hat. Durch die Hohlräume gelangt aufgrund der Kapillarwirkung Flüssigkeit aus dem Behälter in das Innere der Holzstäbchen und verdunstet an der sich nicht in der Flüssigkeit befindlichen Oberfläche der Holzstäbchen. Hierdurch gelangen die in der Flüssigkeit enthaltenen Essenzen in die Luft und beduften beispielsweise den Raum, in dem sich die Vorrichtung befindet.

Wenngleich mittels der bekannten Vorrichtung auch eine Beduftung von Räumen möglich ist, so hat sie dennoch den Nachteil, dass die Verdunstung der Flüssigkeit recht langsam vor sich geht, sowie die Holzstäbchen sichtbar sind, was das Aussehen der Vorrichtung beeinträchtigt.

Es ist Aufgabe der Erfindung, eine eingangs genannte Vorrichtung derart auszubilden, dass sie eine höhere Verdunstung pro Zeiteinheit hat, wobei sie ein formschönes Äußeres hat.

Die Lösung dieser Aufgabe ergibt sich aus den Merkmalen des kennzeichnenden Teils des Anspruchs 1. Vorteilhafte Weiterbildungen der Erfindung ergeben sich aus den Unteransprüchen.

Gemäß der Erfindung ist eine Vorrichtung zum Verdunsten von Essenzen aufweisenden Flüssigkeiten wie ätherische Öle, mit einem Behälter, in den die Flüssigkeit einbringbar ist, sowie einem Verdunsterelement, mittels dem die Flüssigkeit aus dem Behälter entnehmbar und an die Umgebungsluft abgebbar ist, dadurch gekennzeichnet, dass das Verdunsterelement ein saugfähiges flächiges Element aufweist.

Dadurch, dass das Verdunsterelement ein saugfähiges flächiges Element aufweist, wird auf einfache Weise erreicht, dass pro Zeiteinheit eine große Menge an Flüssigkeit verdunsten kann. Denn, da die verdunstete Flüssigkeit maßgeblich von der Oberfläche des Verdunsterelements abhängt, erhöht sich bei einer Vergrößerung der Oberfläche des Verdunsterelements die Menge der verdunsteten Flüssigkeit. Darüber hinaus lässt sich das flächige Element derart ausbilden, dass es wie das Blatt einer Pflanze, insbesondere einer Blume, aussieht. So könnte das flächige Element derart gestaltet sein, dass es beispielsweise wie die Blume aussieht, deren Essenzen in der Flüssigkeit enthalten sind. Hierdurch erhält man auf einfache Weise die Information, welcher Duft durch die Vorrichtung abgegeben wird, wodurch der von der Vorrichtung ausgehende optische Eindruck in Einklang mit dem von der Vorrichtung ausgehenden Duft steht. Des Weiteren ist die so erhaltene Information beim Erwerb der Vorrichtung von Vorteil.

In vorteilhafter Weise besteht das flächige Element aus einem Funktionsfaserstoff wie beispielsweise Modal. Gut eignet sich auch Viskose oder Baumwolle. Hierdurch wird erreicht, dass es relativ unempfindlich gegen Beschädigungen ist, ohne dass sich hierdurch die Wirksamkeit verringert.

Bei einer weiteren Ausführungsform der Erfindung ist vorgesehen, dass das flächige Element mit einem Transportelement verbunden ist, welches saugfähig ist und sich in das Innere des Behälters erstreckt. Wird das Transportelement aus einem Material hergestellt wird, welches eine sehr hohe Saugfähigkeit beziehungsweise Kapillarwirkung hat, lässt sich hierdurch die Wirksamkeit der Vorrichtung verbessern. Da sich das Transportelement zum überwiegenden Teil im Inneren des Behälters befindet, ist es optisch im Wesentlichen nicht wahrnehmbar, weshalb auf das Aussehen des Transportelements keine Rücksicht genommen werden braucht.

Ein Transportelement, welches aus einem Vlies besteht, hat sich als sehr vorteilhaft erwiesen. Es kann aber auch aus einem anderen gut saugfähigen Material wie beispielsweise Textil bestehen.

Bei einer weiteren besonderen Ausführungsform der Erfindung ist vorgesehen, dass das Transportelement mit einem Trägerelement verbunden ist, wobei das Trägerelement vorzugsweise aus einem relativ steifen Material besteht. Hierdurch lässt sich ein Transportelement aus einem sehr weichen Material herstellen und dennoch problemlos in den Behälter einbringen.

In vorteilhafter Weise ist das Transportelement mit einem Beutel umschlossen. Hierdurch lässt sich einerseits ein Transportelement verwenden, welches keine feste Struktur hat. Andererseits wird das Transportelement durch den Beutel geschützt und in Form gehalten.

Das flächige Element kann in vorteilhafter Weise einen Bereich aufweisen, in dem es schnurförmig ausgebildet ist. Besonders vorteilhaft hierbei ist es, wenn sich der schnurförmige Bereich in das Innere des Behälters erstreckt. Hierdurch lässt sich das Transportelement mit dem flächigen Element einstückig ausbilden. Das heißt, das flächige Element kann an einer Stelle beispielsweise so verzwirbelt werden, dass ein schnurförmiger Bereich entsteht. Befindet sich der schnurförmige Bereich in der Flüssigkeit, dient er als Transportelement und versorgt das übrige vorhandene flächige Element mit Flüssigkeit.

Weitere Einzelheiten, Merkmale und Vorteile der vorliegenden Erfindung ergeben sich aus der nachfolgenden Beschreibung eines besonderen Ausführungsbeispiels unter Bezugnahme auf die Zeichnung.

Es zeigt:
- Fig. 1: eine erste Ausführungsform einer erfindungsgemäßen Vorrichtung in schematischer Darstellung und
- Fig. 2: eine zweite Ausführungsform einer erfindungsgemäßen Vorrichtung in schematischer Darstellung.

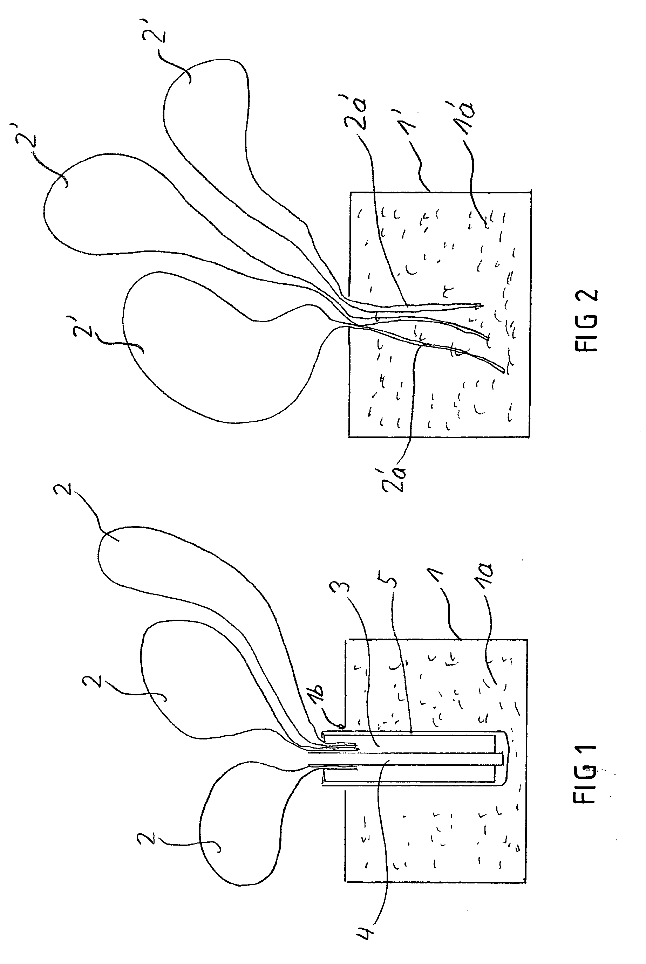

Wie Fig. 1 entnommen werden kann, befindet sich in einem Behälter 1 eine Flüssigkeit 1a, welche ein nach Rosen duftendes ätherisches Öl aufweist. Durch eine Öffnung 1b des Behälters 1 erstreckt sich in die Flüssigkeit 1a ein Transportelement 3, welches aus einem Vlies besteht und um ein als Holzstab 4 ausgebildetes Trägerelement gewickelt ist. Dadurch, dass das Transportelement 3 aus Vlies besteht, wird die Sättigung der Duftstoffe sehr viel später erreicht als bei Transportelementen aus Holz. Das Transportelement 3 sowie das Trägerelement 4 befinden sich in einem flüssigkeitsdurchlässigen Beutel 5. An den sich im Bereich der Öffnung 1b befindlichen Ende des Transportelements 3 ist das Transportelement 3 mit mehreren aus Modal bestehenden flächig ausgebildeten Verdunsterelementen verbunden. Zur Verbindung der Verdunsterelemente 2 mit dem Transportelement 3 können Transportelement 3 und die entsprechenden Bereiche der Verdunsterelemente 2 mit einer Schnur umwickelt sein.

Die Verdunsterelemente 2 sind als Blätter einer Rose ausgebildet, wodurch die Vorrichtung ein formschönes Äußeres enthält und das Aussehen der Verdunsterelemente 2 mit den in der Flüssigkeit 1a enthaltenen Essenzen übereinstimmt. Das heißt, die als Rosenblätter ausgebildeten Verdunsterelemente 2 geben den Duft einer Rose ab.

Die in Fig. 2 dargestellte Ausführungsform der erfindungsgemäßen Vorrichtung hat im Wesentlichen denselben Aufbau wie die in Fig. 1 dargestellte Ausführungsform der erfindungsgemäßen Vorrichtung. Gleiche Elemente sind daher mit denselben Bezugszeichen versehen; zur Unterscheidung weisen sie jedoch einen Strich auf.

Im Unterschied zu der in Fig. 1 dargestellten Ausführungsform sind die flächigen Elemente 2' nicht mit einem separaten Transportelement verbunden. Stattdessen sind die flächigen Elemente 2' an einer Stelle so verzwirbelt, dass ein schnurförmiger Bereich 2a' entsteht. Der schnurförmige Bereich 2a' befindet sich in der Flüssigkeit 1', sodass die Flüssigkeit 1' über den schnurförmigen Bereich 2a' in das flächige Elemente 2' gelangt und dort verdunstet.

## Patentansprüche

1. Vorrichtung zum Verdunsten von Essenzen aufweisenden Flüssigkeiten (1a; 1a') wie ätherische Öle, mit einem Behälter (1; 1'), in den die Flüssigkeit (1a; 1a') einbringbar ist, sowie einem Verdunsterelement (2, 3; 2', 2a'), mittels dem die Flüssigkeit (1a; 1a') aus dem Behälter (1; 1') entnehmbar und an die Umgebungsluft abgebbar ist,
**dadurch gekennzeichnet,**
**dass** das Verdunsterelement (2, 3; 2', 2a') ein saugfähiges flächiges Element (2; 2') aufweist.

2. Vorrichtung nach Anspruch 1,
**dadurch gekennzeichnet,**
**dass** das flächige Element (2; 2') aus einem Funktionsfaserstoff besteht.

3. Vorrichtung nach Anspruch 1 oder 2,
**dadurch gekennzeichnet,**
**dass** das flächige Element (2; 2') mit einem Transportelement (3; 2a') verbunden ist, welches saugfähig ist und sich in das innere des Behälters (1; 1') erstreckt.

4. Vorrichtung nach Anspruch 3,
**dadurch gekennzeichnet,**
**dass** das Transportelement aus einem Vlies besteht.

5. Vorrichtung nach Anspruch 3 oder 4,
**dadurch gekennzeichnet,**
**dass** das Transportelement (3) mit einem Trägerelement (4) verbunden ist.

6. Vorrichtung nach einem der Ansprüche 3 bis 5,
**dadurch gekennzeichnet,**
**dass** das Transportelement (3) mit einem Beutel (5) umschlossen ist.

7. Vorrichtung nach einem der Ansprüche 1 bis 6,
**dadurch gekennzeichnet,**
**dass** das flächige Element (2') einen Bereich (2a') aufweist, in dem es schnurförmig ausgebildet ist.

8. Vorrichtung nach Anspruch 7,
**dadurch gekennzeichnet,**
**dass** sich der schnurförmige Bereich (2a') in das Innere des Behälters (1') erstreckt.
